# EUROPÄISCHE PATENTSCHRIFT

(11) **EP 1 117 636 B1**
(45) Veröffentlichungstag und Bekanntmachung des Hinweises auf die Patenterteilung: **11.12.2002**
(21) Anmeldenummer: 99948764.8
(22) Anmeldetag: 16.09.1999
(51) Int. Cl.: C07C 249/08, C07C 251/34, C07C 251/60

(54) **VERFAHREN ZUR HERSTELLUNG VON TRION-BIS(OXIMETHER) DERIVATEN SOWIE DAMIT ERHÄLTLICHE TRION-MONO- UND TRION-BIS(OXIMETHER) DERIVATE**
METHOD FOR PREPARING TRION-BIS(OXIME ETHER) DERIVATIVES ANDRION-MONO AND TRION-BIS(OXIME ETHER) DERIVATIVES OBTAINED THEREWITH
PROCEDE DE FABRICATION DE DERIVES DE TRION-BIS(OXIME-ETHER) ET DERIVES DE TRION-MONO- ET DE TRION-BIS(OXIME-ETHER) AINSI OBTENUS

(30) Priorität: 30.09.1998 DE 19844919
(43) Veröffentlichungstag der Anmeldung: 25.07.2001
(73) Patentinhaber: BASF AKTIENGESELLSCHAFT, 67056 Ludwigshafen (DE)
(72) Erfinder: GROTE, Thomas, D-67105 Schifferstadt (DE); WOLF, Bernd, D-67136 Fussgönheim (DE); RACK, Michael, D-69123 Heidelberg (DE); GÖTZ, Roland, D-68809 Neulu heim (DE); GYPSER, Andreas, D-68159 Mannheim (DE); STEINMETZ, Adrian, D-68165 Mannheim (DE); SAUTER, Hubert, D-68305 Mannheim (DE); KEIL, Michael, D-67251 Freinsheim (DE); MAYER, Horst, D-67069 Ludwigshafen (DE)
(74) Vertreter: Münch, Volker, Dr.
(86) Internationale Anmeldenummer: EP9906862
(87) Internationale Veröffentlichungsnummer: WO00018726

(56) Entgegenhaltungen:
- EP-A- 0 940 388
- WO-A-97/15552
- US-A- 4 435 421

## Beschreibung

Die vorliegende Erfindung betrifft ein Verfahren zur Herstellung von Trion-bis(oximether)derivaten der Formel I, 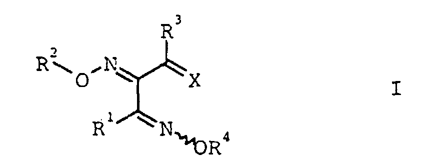 in der die Substituenten die folgende Bedeutung haben:
- R¹,R³: unsubstituiertes, teilweise oder vollständig halogeniertes C₁-C₆-Alkyl oder C₃-C₆-Cycloalkyl;
- R²,R⁴: unsubstituiertes C₁-C₄-Alkyl oder durch C₂-C₄-Alkenyl, C₂-C₄-Alkinyl oder Phenyl substituiertes Methyl und
- X: Sauerstoff oder N-OH.

Außerdem betrifft die Erfindung mit diesem Verfahren erhältliche Ketale der Formel III,

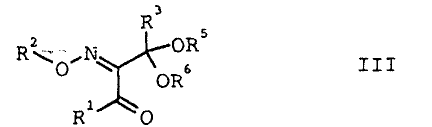

Bisoximetherketale der Formel IV sowie

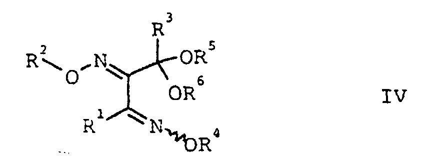

Bisoximetherketone der Formel Ia und Bisoximetheroxime der Formel Ib sind interessante Zwischenprodukte zur Herstellung der aus WO-A 97/15552 bekannten Pflanzenschutzwirkstoffe.

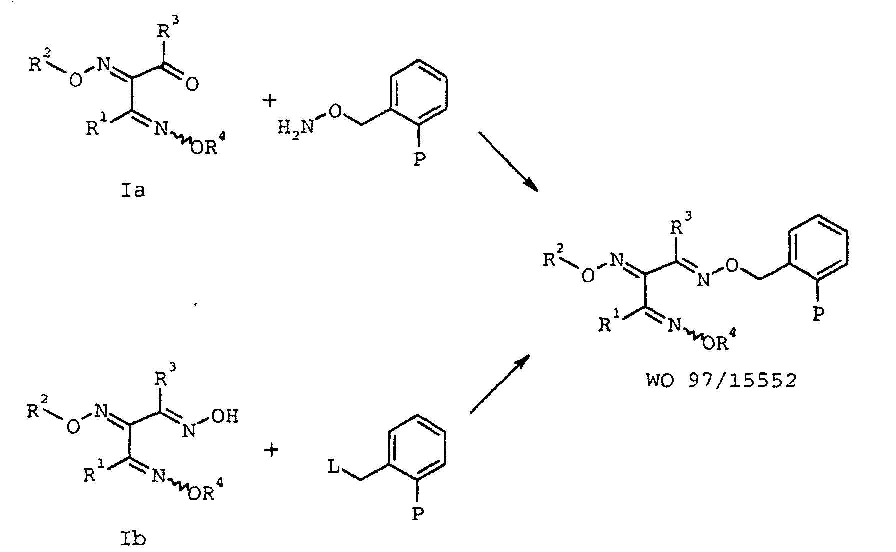

Im Stand der Technik befassen sich nur wenige Dokumente mit der Synthese von Bisoxim- bzw. Trisoximderivaten von vicinalen Triketonen. Ferner weisen einige der z.T. älteren Dokumente ungenaue bzw. falsche Strukturangaben auf (Gazz. Chim. Ital., 67 (1937) S. 388; Gazz. Chim. Ital., 52 (1922) S. 289). Erst modernere analytische Methoden erlauben die Strukturaufklärung der komplexen Stoffmischungen, die beispielsweise bei der Umsetzung von 3-(Hydroxyimino)pentan-2,4-dion mit Hydroxylamin entstehen: neben den (E,E,E)- und (E,Z,E)-Isomeren des Pentan-2,3,4-triontrisoxims werden ringgeschlossene Furoxane und Isoxazole gebildet (J. Chem. Soc., Perkin Trans. II (1987) S. 523). Aufgrund der gebildeten cyclischen Nebenkomponenten und der falschen Regio- und Stereochemie kommen die durch Umsetzung von Triketonen und Hydroxylamin erhaltenen Stoffgemenge für den Aufbau der Trionbis(oximether)derivate Ia und Ib nicht in Frage.

Ein gezielter Aufbau der Bisoximetheroxime Ib wird in der WO 97/15552 beschrieben.

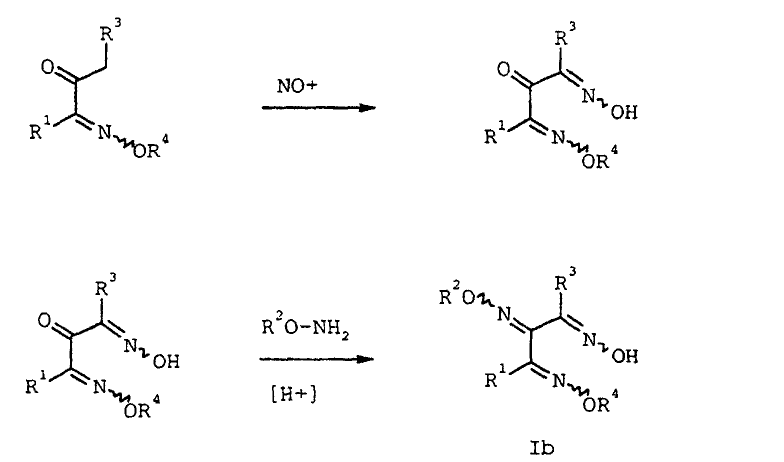

Ein Nachteil dieser Synthesesequenz ist, daß die zentrale Oximetherfunktion (R²O-N=C) erst im letzten Schritt aufgebaut wird. Da sich die beiden Substituenten am zentralen Kohlenstoffatom (R¹-C=NOR⁴ und R³-C=NOH) hinsichtlich ihrer sterischen Beanspruchung wenig unterscheiden, verläuft die Oximierung nicht stereoselektiv und es entstehen hinsichtlich der Bindung R²O-N Isomerengemische, die sich nur schwer auftrennen lassen.

Die Aufgabe der vorliegenden Erfindung bestand folglich darin, ein Verfahren zu finden, mit Hilfe dessen sich Verbindungen der Formel Ia und Ib gezielt aufbauen lassen und das zudem die gewünschten Isomeren dieser Verbindungen auf direktem Wege, d.h. ohne eine Isomerentrennung, liefert.

Demgemäß wurde das eingangs erwähnte Verfahren gefunden, daß dadurch gekennzeichnet ist, daß man
1) ein Dion der Formel II, 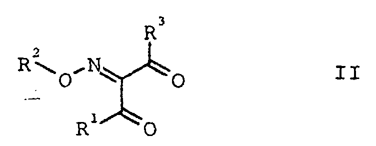 in der die Substituenten R¹, R² und R³ die vorgenannte Bedeutung besitzen, mit einem Alkohol oder Diol in Gegenwart einer Säure zum Ketal der Formel III, 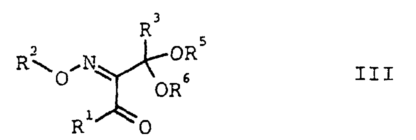 in der die Substituenten R⁵ und R⁶ C₁-C₆-Alkyl, Benzyl oder C₁-C₃-Haloalkyl bedeuten oder R⁵ und R⁶ zusammen mit dem Kohlenstoff- und den beiden Sauerstoffatomen der Ketalfunktion einen Ring A bilden, 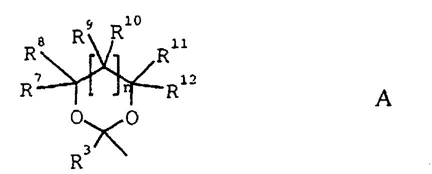 wobei die Substituenten und der Index n die folgende Bedeutung haben:
   - R⁷,R⁸,R¹¹,R¹²: Wasserstoff, Halogen, C₁-C₄-Alkyl, C₁-C₃-Haloalkyl, C₁-C₄-Alkoxymethyl, C₂-C₄-Alkenyl, C₂-C₄-Alkinyl oder Phenyl, wobei letzeres durch Nitro oder Halogen substituiert sein kann;
   - R⁹,R¹⁰: eine der für R⁷, R⁸, R¹¹ oder R¹² gegebene Bedeutung sowie R⁹ und R¹⁰ gemeinsam eine exo-Methylen-Gruppe oder eine Carbonylgruppe und
   - n: 0, 1 oder 2,
   umsetzt,
2) das so erhaltene Ketal III
   a) mit einem Alkoxyamin der Formel R⁴O-NH₂, wobei R⁴ die vorgenannte Bedeutung besitzt, oder einem seiner Säureadditionssalze, oder
   b) mit Hydroxylamin oder dessen Säureadditionssalz und anschließender Alkylierung mit einem Alkylierungsmittel R⁴-L¹, wobei R⁴ die vorgenannte Bedeutung besitzt und L¹ für eine nukleophil austauschbare Abgangsgruppe steht, in das Bisoximetherketal IV, 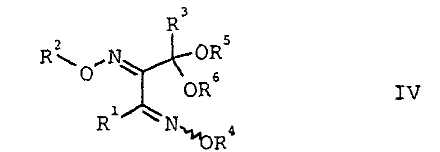
   wobei die Substituenten R¹ bis R⁶ die vorgenannte Bedeutung besitzen, überführt und
3) das auf diese Weise erhaltene Bisoximetherketal IV in Gegenwart von Säure
   a) zum Bisoximetherketon Ia, 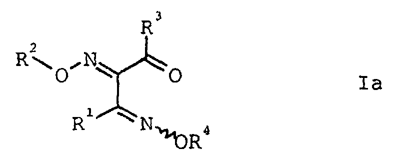 hydrolysiert oder
   b) mit Hydroxylamin oder dessen Säureadditionssalz zum Bisoximetheroxim Ib, 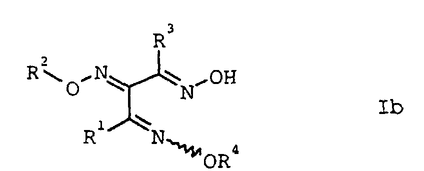 aminiert.

Mit dem erfindungsgemäßen Verfahren lassen sich je nach Ausgestaltung der Stufe 3) gezielt Verbindungen der Formel Ia bzw. Ib synthetisieren. Ein weiterer Vorteil des Verfahrens besteht darin, daß die Verbindungen Ia und Ib hinsichtlich der zentraler Oximether-Einheit in isomerenreiner Form anfallen.

In Schema 1 ist eine besondere Ausführungsform des Verfahrens dargestellt.

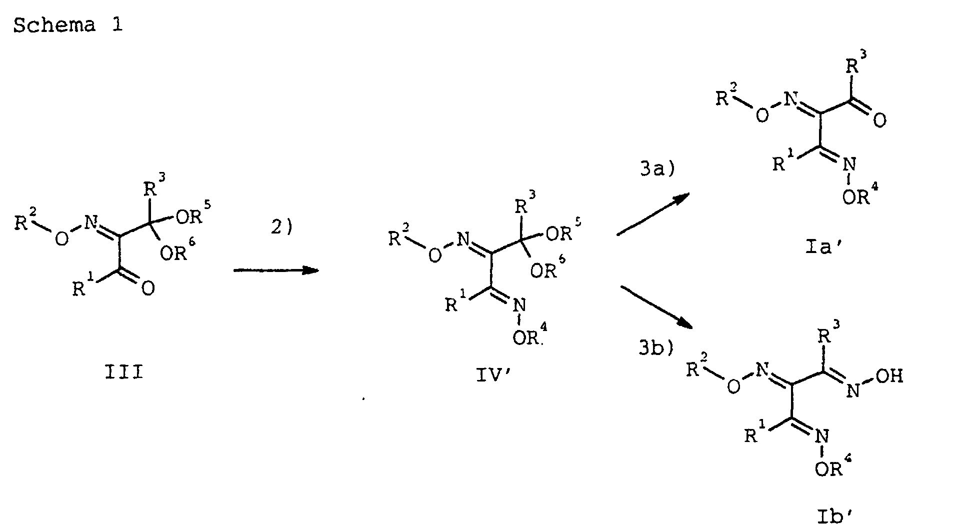

Durch geeignete Reaktionsführung können vorzugsweise das E,E-Isomere Ia' und E,Z,E-Isomere Ib' via der Bisoximetherketale IV' erhalten werden (s. Schema 1):
- in Stufe 1) werden Diole wie beispielsweise Ethylenglykol, 1,3-Propandiol oder vorzugsweise 2,2-Dimethyl-1,3-propandiol eingesetzt, die cyclische Ketale III liefern.
- die Oximierungsstufe wird nach Variante 2a) durchgeführt.. Im Einzelnen wird das Ketal III mit einem Säureadditionssalz des Alkoxy-amins R⁴O-NH₂ bei Temperaturen von 20 bis 65°C umgesetzt und die bei der Reaktion freiwerdende Säure durch Zusatz von Basen mindestens teilweise gebunden.
- in Stufe 3a)/3b) wird die Hydrolyse/Aminolyse bei einem pH von 0,5 bis 1,5 gestartet und bei Temperaturen von 20 - 40°C durchgeführt.

Geht man umgekehrt beispielsweise von Dimethylketal IIIa (R⁵, R⁶ = Methyl) aus, das bei Temperaturen größer 40°C hydrolysiert (Stufe 3a) bzw. aminiert (Stufe 3b) wird, so steigen die Anteile des Z-Isomeren Ia'' bzw. Ib'' in der Reaktionsmischung in der Regel an.

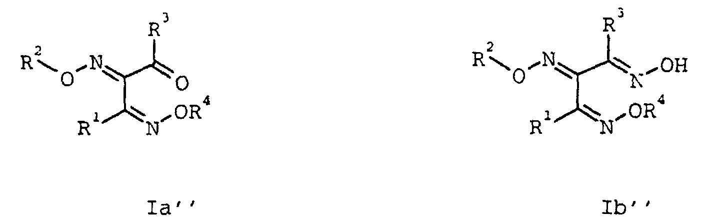

Die einzelnen verfahrensschritte werden im folgenden näher erläutert.

### 1) Ketalbildung

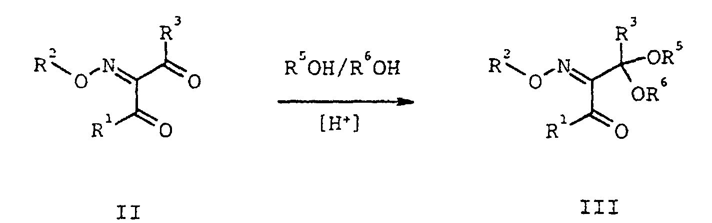

Die Ketalbildung kann im allgemeinen mit C₁-C₆-Alkanolen wie beispielsweise Methanol, Ethanol, n-Propanol, i-Propanol, n-Butanol, i-Butanol, s-Butanol, n-Pentanol, mit Benzylalkohol oder mit C₁-C₃-Haloalkylalkoholen wie beispielsweise 2,2,2-Trichlorethanol durchgeführt werden. Besonders geeignet sind Diole wie beispielsweise Brenzcatechin, Ethylenglykol (1,2-Ethandiol), 1-(2-Nitrophenyl)-1,2-ethandiol, Hex-5-en-1,2-diol, 1,3-Propandiol, 2,2-Dimethyl-1,3-propandiol, 3-Brom-1,2-propandiol, 2-exo-Methylen-1,3-propandiol, 2,2-Dibrom-1,3-propandiol, 1,4-Butandiol, 1,4-Dimethoxy-2,3-butandiol. Insbesondere eignen sich sterisch anspruchsvolle Diole wie 1,3-Propandiol und 2,2-Dimethyl-1,3-propandiol.

Die Ketalbildung wird in der Regel in Gegenwart von Säuren wie BF₃ x Et₂O (Lewis Säure) oder vorzugsweise Brönstedt Säuren wie Schwefelsäure, Chlor-, Brom- oder Iodwasserstoff, Perchlorsäure, Orthophosphorsäure, Polyphosphorsäure, p-Toluolsulfonsäure, p-Dodecylbenzolsulfonsäure oder Camphersulfonsäure durchgeführt. Bevorzugt ist der Einsatz von p-Toluolsulfonsäure oder Schwefelsäure.

Die Säure wird gewöhnlich in katalytischen Mengen von 0,05 bis 2 Mol% und vorzugsweise in 0,5 bis 1 Mol% in Bezug auf das Dion II eingesetzt.

Die Reaktionstemperatur hängt im allgemeinen von der Art des eingesetzten Alkohols ab und beträgt in der Regel 20 bis 150°C und vorzugsweise 60 bis 110°C. Beim Einsatz von Diolen hat sich in vielen Fällen eine Temperatur von 60 bis 90°C als vorteilhaft herausgestellt.

Das bei der Reaktion entstehende Wasser wird gewöhnlich aus der Reaktionsmischung entfernt. Hierbei stehen die im Stand der Technik beschriebenen Methoden zur Verfügung (s. beispielsweise Organikum, Barth Verlagsgesellschaft, Leipzig).

Das Reaktionswasser kann zum einen mit Hilfe wasserentziehender Mittel wie beispielsweise Orthoester entfernt werden. Der Orthoester wie beispielsweise Trimethylorthoformiat wird in der Regel in einer Konzentration von 1 bis 1,5 Moläquivalenten eingesetzt. Die Reaktionszeit beträgt im allgemeinen 0,5 bis 3 Stunden.

Anderseits hat sich bewährt, das Reaktionswasser durch Wasserauskreisung mit Schleppmitteln wie Toluol oder Cyclohexan zu entfernen. Anhand der Menge des abgeschiedenen Wassers kann der Endpunkt der Reaktion leicht ermittelt werden. In manchen Fällen ist es von Vorteil, wenn die Reaktion bei reduziertem Druck durchgeführt wird.

Als Lösungsmittel dient vorzugsweise der für die Ketalisierung benötigte Alkohol, der dann im allgemeinen im Überschuß eingesetzt wird. Gute Ergebnisse ließen sich beispielsweise mit 1-10 Moläquivalenten an Diol erzielen. Wird die Ketalisierung mittels Wasserauskreisung in Gegenwart eines Schleppmittels durchgeführt, kann die Diolmenge im allgemeinen auf 1-3 Moläquivalente reduziert werden. Als Lösungsmittel sind weiterhin Kohlenwasserstoffe wie beispielsweise Toluol oder Cyclohexan, Halogenkohlenwasserstoffe wie Chlorbenzol oder Methylenchlorid, Amide wie Dimethylformamid und Ether wie Diethylether oder Dioxan geeignet.

Die Aufarbeitung der Reaktionsgemische erfolgt beispielsweise durch Extraktion mit einem unpolaren Lösungsmittel wie einem Ether, Halogenkohlenwasserstoff oder insbesondere einem Kohlenwasserstoff wie Cyclohexan. Nach dem Abtrennen der wäßrigen Phase kann die organische Phase in der Regel direkt in die darauffolgende Oximierungsstufe eingesetzt werden. In vielen Fällen ist es nicht einmal notwendig einen Austausch des Lösungsmittels vorzunehmen.

Die Dione der Formel II sind aus der Literatur bekannt oder können nach literaturbekannten Methoden hergestellt werden [vgl. indian J. Chem. B, (1991) 749-753; Bull. Acad. Sci. USSR Div. Chem. Sci. (Engl. Transl.) 28, (1979) 121-128; EP-A 416 857].

Die Dione II können insbesondere nach der im folgenden näher erläuterten Verfahrensweise hergestellt werden .

Die 1,3-Diketone der Formel V 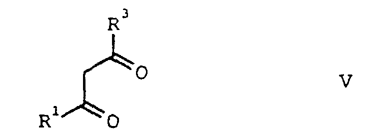 werden durch Nitrosierung in Verbindungen der Formel VI, 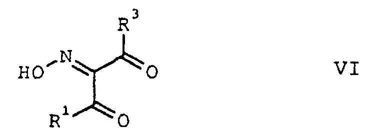 wobei die Substituenten R¹ und R³ in den Formeln V und VI die in Anspruch 1 angegeben Bedeutung haben, übergeführt.

Die Nitrosierung wird üblicherweise mit Natriumnitrit in Gegenwart einer Carbonsäure oder Mineralsäure durchgeführt. Besonders geeignet sind Essigsäure, Salzsäure und insbesondere Schwefelsäure.

Im allgemeinen wird die Nitrosierung bei Temperaturen von -10 bis 60°C und insbesondere bei 10 bis 20°C durchgeführt.

In der Regel wird bei einem pH von 2 bis 6 und insbesondere bei einem pH von 4 bis 5 nitrosiert.

Als besonders vorteilhaft haben sich die folgenden Verfahrensvarianten herausgestellt: i) das 1,3-Diketon V wird in wäßriger Natriumnitrit-Lösung vorgelegt. Anschließend wird die Säure bei einem pH von 4 bis 5 zugetropft; ii) das 1,3-Diketon V wird in Wasser vorgelegt und die Säure sowie die wäßrige Natriumnitrit-Lösung werden simultan bei einem pH von 4 bis 5 zudosiert.

Weiterhin kann es von Vorteil sein, zu Beginn oder am Ende der Reaktion ein organisches Lösungsmittel zuzusetzten, in dem sich Verbindung VI löst. Die somit erhaltenen Lösungen lassen sich unmittelbar in den darauf folgenden Alkylierungsschritt einsetzen. Eine Zwischenisolierung der thermisch und gegen Hydrolyse instabilen Verbindung VI läßt sich auf diese Weise vermeiden. Unter Umständen kann es weiterhin vorteilhaft sein, das zur Extraktion von VI benutzte Lösungsmittel gegen ein für die Alkylierung besser geeignetes Lösungsmittel auszutauschen. Für die Extraktion eigenen sich insbesondere aprotische, mit Wasser ggf. teilweise mischbare Lösungsmittel, beispielsweise Halogenkohlenwasserstoffe wie Methylenchlorid, Carbonsäureester wie Essigester oder Ether wie Methyl-tert.-butylether.

Die Alkylierung von VI zu den Dionen II kann beispielsweise in Alkoholen wie Methanol, Halogenkohlenwasserstoffe wie Methylenchlorid, Carbonsäureester wie Essigester oder Ether wie Methyltert.-butylether erfolgen. Insbesondere geeignet sind Ketone wie Aceton und Amide wie Dimethylformamid oder N-Methylpyrrolidon.

Als Alkylierungsmittel kommen beispielsweise Alkylhalogenide, Tosylate und Dialkylsulfate in Frage. Besonders geeignet sind Dialkylsulfate der Formel VII,

(R²O)₂SO₂ VII

wobei der Substituent R² die in Anspruch 1 angegeben Bedeutung hat.

Die Alkylierung wird üblicherweise in Gegenwart von Basen wie Alkali- oder Erdalkalihydroxiden, Alkali- oder Erdalkalicarbonaten, Alkali- oder Erdalkalialkoholaten oder tertiären Amiden durchgeführt.

Die Reaktionstemperatur beträgt in der Regel -20 bis 100°C und vorzugsweise -10 bis 35°C und insbesondere 0 bis 25°C.

Üblicherweise wird das Lösungsmittel und die Base vorgelegt und anschließend Verbindung VI und das Alkylierungsmittel gleichzeitig oder nacheinander zudosiert.

### 2) Oximierung

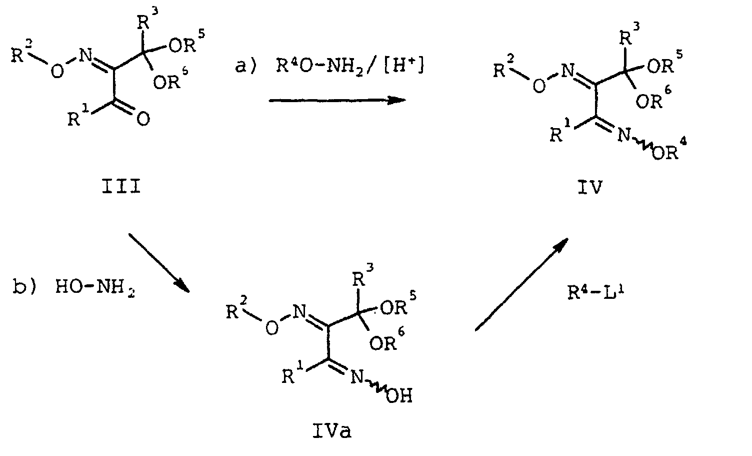

2a) Das Alkoxyamin R⁴O-NH₂ wird entweder in Form eines Säureadditionssalzes oder als freie Base eingesetzt, wobei letztere durch Zugabe einer starken Base aus dem Salz freigesetzt werden kann.

Vorzugsweise werden die Säureadditionssalze des Alkoxyamins eingesetzt. Für die Herstellung der Säureadditionssalze eignen sich alle gängigen Säuren. Im folgenden seien lediglich einige beispielhaft aufgeführt: Carbonsäuren wie Essig- oder Propionsäure, Dicarbonsäuren wie Oxal- oder Bernsteinsäure, Mineralsäuren wie Phosphor- oder Kohlensäure und insbesondere Salzsäure oder Schwefelsäure.

Werden die Säureadditionssalze des Alkoxyamins eingesetzt, so ist es in der Regel von Vorteil eine Base zuzusetzen, um die bei der Reaktion freiwerdende Säure zu binden. In vielen Fällen hat sich ein pH von 2 bis 5 unc insbesondere von 3 bis 4 für die Oximierung als vorteilhaft herausgestellt.

In der Regel werden 1 bis 2,5 Moläquivalente einer Base zugesetzt. Als Basen sind insbesondere Pyridine, Trialkylamine, Natriumhydroxid, Natriumacetat und Natriummethylat geeignet. Bei der Verwendung von Natriumacetat wird üblicherweise noch Eisessig zugesetzt.

Natürlich kann umgekehrt auch das Alkoxyamin als freie Base eingesetzt werden und zur Einstellung des obenerwähnten pH-Bereichs eine der genannten Säuren verwendet werden.

Als Lösungsmittel können beispielsweise die in der vorhergehenden Stufe beschriebenen eingesetzt werden. Darüberhinaus kommen auch Carbonsäuren wie Essigsäure, Ether wie Tetrahydrofuran, Diethylether, Methyl-tert.-bucylether oder auch Wasser/Pyridin-Gemische in Frage. Insbesondere sind Alkohole wie Methanol, Ethanol, n-Propanol oder i-Propanol geeignet.

Als vorteilhaft hat sich ferner erwiesen, das in der Ketalisierung verwendete Lösungsmittel, bzw. das nach der Aufarbeitung der Ketale III vorliegende Lösungsmittelgemisch auch für die Oximierungsstufe zu verwenden. Gegebenenfalls kann es angebracht sein, dem Gemisch noch weitere Lösungsmittel zuzusetzen. Die Stufen 1) und 2) lassen sich demnach als Eintopfvariante durchführen.

Die Reaktionstemperatur beträgt in der Regel -20 bis 150°C und vorzugsweise 0 bis 100°C und insbesondere 20 bis 65°C.

2b) Die unter 2a) geschilderte Verfahrensweise, läßt sich auch zweistufig, durch zunächst Umsetzung des Ketals III mit Hydroxylamin oder dessen Säureadditionssalz und anschließender Alkylierung mit R⁴-L¹, durchführen. Hinsichtlich der Reaktionsführung gelten die obengenannten Äußerungen.

Die Aufarbeitung der Reaktionsmischung erfolgt vorzugweise, wie in der vorangegangenen Stufe beschrieben, mittels extraktiver Methoden.

### 3) Ketalspaltung: (a) Hydrolyse und (b) Aminierung

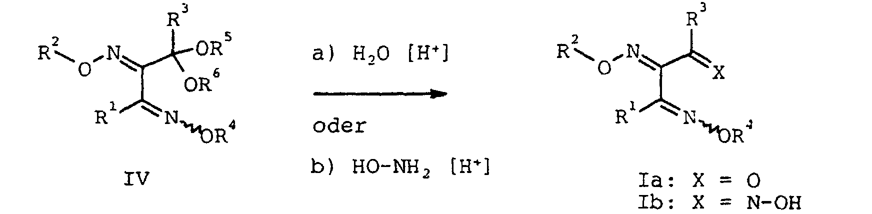

Die Ketalspaltung erfolgt üblicherweise im Sauren. Dabei hat sich ein pH von 0 bis 2 und vorzugsweise von 0,5 bis 1,5 als vorteilhaft erwiesen.

Die Einstellung des genannten pH Bereichs kann mit jeder gängigen Säure erfolgen. Als geeignet haben sich beispielsweise Essigsäure, Salzsäure oder Schwefelsäure erwiesen.

Die Ketalspaltung kann mit oder ohne den Zusatz eines Lösungsmittels durchgeführt werden. Als vorteilhaft hat sich der Einsatz von organischen Lösungsmitteln erwiesen, die im genannten pH Bereich stabil sind (z.B. Essigester). Auch kann es von Vorteil sein ein mit Wasser/Säure einphasig mischbares Lösungsmittel zu verwenden. Hier bieten sich insbesondere Alkohole wie beispielsweise Methanol an. Geeigneterweise kann die Ketalspaltung beispielsweise in Wasser/Methanol/Eisessig (ein geeignetes Mischungsverhältnis ist beispielsweise: 1/1/0,2) bzw. Essigester/Wasser-Mischungen durchgeführt werden.

Die Aminolyse zu den Verbindungen Ib wird unter den für die Ketalspaltung aufgeführten Bedingungen, lediglich in Gegenwart von Hydroxylamin bzw. dessen Säureadditionssalz durchgeführt. Zur Herstellung der Säureadditionssalze kommen alle gängigen Säuren in Frage. Als besonders vorteilhaft hat sich Salzsäure bzw. Schwefelsäure erwiesen.

Das Hydroxylamin bzw. dessen Säureadditionssalz wird im allgemeinen in einem Verhältnis von 1 bis 2 und vorzugsweise 1 bis 1,3 Moläquivalenten in Bezug auf das Bisoximetherketal IV eingesetzt.

Die Reaktionstemperatur beträgt im allgemeinen 0 bis 150°C. Zur Herstellung der Isomeren Ia' und insbesondere Ib' haben sich niedrige Reaktionstemperaturen von 20 bis 40°C als besonders vorteilhaft herausgestellt. Bei hohen Reaktionstemperaturen (>40°C) steigt in der Regel der Anteil der Isomeren Ia'' und Ib'' an.

Die Aufarbeitung der Reaktionsmischungen erfolgt vorzugsweise, wie in den beiden vorangegangenen Stufen beschrieben, auf extraktivem Wege.

Die Verbindungen der Formel Ib können beispielsweise über ihr Natriumsalz gereinigt werden. Durch Zugabe einer Base lassen sich die Oxime in das entsprechende Salz überführen. Aus dem Salz, das gegebenenfalls abgetrennt oder gereinigt wurde, kann das Bisoximetheroxim Ib durch anschließendes Ansäuern wieder freigesetzt werden.

Mit dem erfindungsgemäßen Verfahren lassen sich insbesondere Ketale der Formel III,

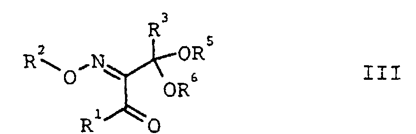

Bisoximetherketale der Formel IV sowie

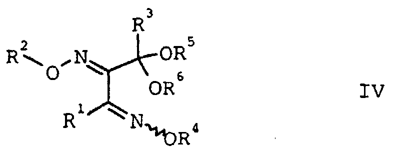

Bisoximetherketone der Formel I 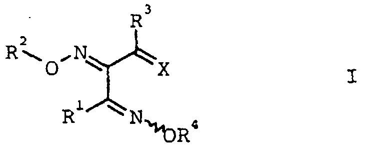 herstellen, in denen die Substituenten jeweils die folgende Bedeutung haben:
- R¹, R³: unsubstituiertes, teilweise oder vollständig halogeniertes C₁-C₆-Alkyl oäer C₃-C₆-Cycloalkyl;
- R², R⁴: unsubstituiertes C₁-C₄-Alkyl oder durch C₂-C₄-Alkenyl, C₂-C₄-Alkinyl oder Phenyl substituiertes Methyl;
- X: Sauerstoff oder N-OH;
- R⁵, R6: C₁-C₆-Alkyl, Benzyl oder C₁-C₃-Haloalkyl oder
- R⁵, R⁶: bilden zusammen mit dem Kohlenstoff- und den beiden Sauerstoffatomen der Ketalfunktion einen Ring A, 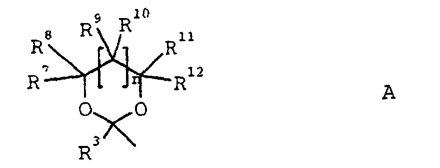 wobei die deutung Substituenten und der Index n die folgende Behaben:
R⁷,R⁸,R¹¹,R¹² Wasserstoff, Halogen, C₁-C₄-Alkyl, C₁-C₃-Haloalkyl, C₁-C₄-Alkoxymethyl, C₂-C₄-Alkenyl, C₂-C₄-Alkinyl oder Phenyl, wobei letzeres durch Nitro oder Halogen substituiert sein kann;
R⁹,R¹⁰ eine der für R⁷, R⁸, R¹¹ oder R¹² gegebene Bedeutung sowie R⁹ und R¹⁰ gemeinsam eine exo-Methylen-Gruppe oder eine Carbonylgruppe und
n 0, 1 oder 2.

Als Zwischenprodukte zur Herstellung der Verbindungen IV (R⁴ ungleich Wasserstoff) können Verbindungen der Formel IV dienen, in denen R⁴ Wasserstoff bedeutet (vgl. Formel IVa).

Bei den zuvor angegebenen Definitionen der Verbindungen I, II und IV wurden für die Reste R¹ bis R¹² Sammelbegriffe verwendet, die für individuelle Aufzählungen der einzelnen Gruppenmitglieder stehen. Die Reste Alkyl, Alkenyl oder Alkinyl können geradkettig oder verzweigt sein.

Die Angabe "teilweise oder vollständig halogeniert" soll zum Ausdruck bringen, daß in den derart charakterisierten Gruppen die Wasserstoffatome zum Teil oder vollständig durch gleiche oder verschiedene Halogenatome ersetzt sein können. Die Bedeutung Halogen steht jeweils für Fluor, Chlor, Brom oder Jod.

Ferner bedeuten beispielsweise:
- C₁-C₄-Alkyl:
   Methyl, Ethyl, Propyl, 1-Methylethyl, Butyl, 1-Methylpropyl, 2-Methylpropyl und 1,1-Dimethylethyl;
- C₁-C₆-Alkyl:
   C₁-C₄-Alkyl, wie voranstehend genannt, sowie Pentyl, 1-Methylbutyl, 2-Methylbutyl, 3-Methylbutyl, 2,2-Dimethylpropyl, 1-Ethylpropyl, Hexyl, 1,2-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1-Ethyl-1-methylpropyl und 1-Ethyl-3-methylpropyl;
- C₁-C₃-Halogenalkyl:
   einen C₁-C₃-Alkylrest wie vorstehend genannt, der partiell oder vollständig durch Fluor, Chlor, Brom und/oder Jod substituiert ist, also z.B. Chlormethyl, Dichlormethyl, Trichlormethyl, Fluormethyl, Difluormethyl, Trifluormethyl, Chlorfluormethyl, Dichlorfluormethyl, Chlordifluormethyl, 2-Fluorethyl, 2-Chlorethyl, 2-Bromethyl, 2-Jodethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl, 2-Fluorpropyl, 3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifluorpropyl, 3,3,3-Trichlorpropyl, 2,2,3,3,3-Pentafluorpropyl, Heptafluorpropyl, 1-(Fluormethyl)-2-fluorethyl, 1-(Chlormethyl)-2-chlorethyl, 1-(Brommethyl)-2-bromethyl;
- C₁-C₄-Alkoxy im Alkoxyteil von C₁-C₄-Alkoxymethyl: Methoxy, Ethoxy, Propoxy, 1-Methylethoxy, Butoxy, 1-Methylpropoxy, 2-methylpropoxy und 1,1-Dimethylethoxy;
- C₂-C₄-Alkenyl: Ethenyl, Prop-1-en-1-yl, Prop-2-en-1-yl, 1-Methylethenyl, But-1-en-1-yl, But-2-en-1-yl, But-3-en-1-yl, 1-Methyl-prop-1-en-1-yl, 2-Methyl-prop-1-en-1-yl, 1-Methylprop-2-en-1-yl und 2-Methyl-prop-2-en-1-yl;
- C₂-C₄-Alkinyl: Ethinyl, 1-Propinyl, 2-Propinyl, 1-Butinyl, 2-Butinyl, 3-Butinyl, 1-Methyl-2-propinyl;
- C₃-C₆-Cycloalkyl: Cyclopropyl, Cyclobutyl, Cyclopentyl, Cyclohexyl.

Im Hinblick auf ihre Eignung als Zwischenprodukte zur Herstellung der aus WO-A 97/15552 bekannten Pflanzenschutzwirkstoffe sind insbesondere die Verbindungen der Formeln I, III und IV mit folgenden Substituenten bevorzugt, wobei die Bevorzugung jeweils für sich allein oder in Kombination zu sehen ist:
- R¹, R³: Methyl, Ethyl, Trifluormethyl oder Trichlormethyl und insbesondere Methyl oder Ethyl;
- R², R⁴: Methyl, Ethyl, Benzyl oder Propargyl und insbesondere Methyl;
- X: Sauerstoff oder N-OH;
- R⁵, R⁶: Methyl, Ethyl, n-Propyl, i-Propyl, n-Butyl, i-Butyl, s-Butyl oder Benzyl und insbesondere
- R⁵, R⁶: bilden zusammen mit dem Kohlenstoff- und den beiden Sauerstoffatomen der Ketalfunktion einen Ring A, 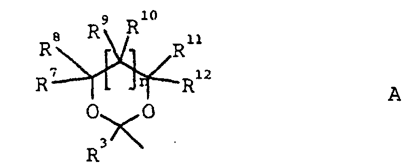 wobei die Substituenten und der Index n die folgende Bedeutung haben:
R⁷,R⁸,R¹¹,R¹² Wasserstoff, Brom oder Methyl und vorzugsweise Wasserstoff oder Methyl;
R⁹,R¹⁰ eine der für R⁷, R³, R¹¹ oder R¹² gegebene Bedeutung und
n 0 oder 1 und insbesondere 1.

Desweiteren sind im Hinblick auf ihre Eignung als Zwischenprodukte zur Herstellung der aus WO-A 97/15552 bekannten Pflanzenschutzwirkstoffe insbesondere die Verbindungen der Formeln IV', Ia' und Ib' bevorzugt.

Insbesondere sind die in den folgenden Herstellungsbeispielen aufgeführten Verbindungen bevorzugt.

### Herstellungsbeispiele

### Herstellung der Dione II (Vorprodukte)

### Pentan-2,3,4-trion-3-oxim

Variante a): In einem Rührbehälter wurden 21 l 20%ige Schwefelsäure und 6 kg (60 mol) Acetylaceton vorgelegt. Es wurde auf ca. 17°C abgekühlt und bei 15 bis 20°C 4,2 kg (60,84 mol) 40,5%-ige, wäßrige Natriumnitrit-Lösung zudosiert. Anschließend wurde noch 20 Minuten bei ca. 17°C nachgerührt. Es wurde mit 25 1 Essigsäureethylester extrahiert. Die organische Phase wurde im Vakuum eingeengt, wobei man 7,42 kg (96% Ausbeute) der Titelverbindung erhielt.

Variante b): Zu einer Mischung aus 500 g (5 mol) Acetylaceton, 1 l Wasser und 1305 g 25%-iger, wäßriger Natriumnitrit-Lösung wurden 1225 g 20%ige Schwefelsäure zudosiert, wobei ein pH von 3 bis 5 und eine Innentemperatur von 25 bis 17°C eingestellt wurde. Das Wertprodukt wurde wie in Variante a) isoliert. Man erhielt 570 g der Titelverbindung (89% Ausbeute).

Variante c): Zu einer Mischung aus 500 g (5 mol) Acetylaceton and 2 l Wasser wurden parallel 490 g (2,5 mol) 50%ige Schwefelsäure und 852 g (5 mol) 40,5%ige wäßrige Natriumnitrit-Lösung dosiert, wobei ein pH von 3,7 bis 4,2 und eine Tempertur von 15 bis 18°C eingestellt wurde. Nach Aufarbeitung wie in Variante a) wurden 588 g der Titelverbindung erhalten (91% Ausbeute).

### Pentan-2,3,4-trion-3-(O-methyloxim)

In einem 20 l Gefäß wurden 4,5 kg (32,6 mol) Kaliumcarbonat in 3,2 l Methyl-tert.-butylether und einem Liter DMF suspendiert. Das Gemisch wurde unter Rühren auf 0 bis -10°C abgekühlt. Anschließend wurde eine Lösung aus 4128 g (32 mol) Pentan-2,3,4-trion-3-oxim, 2 l DMF und 4032 g (32 mol) Diemethylsulfat innerhalb von 2 Stunden bei einer Innentemperatur von <25°C zudosiert. Es wurde 3,5 Stunden bei Raumtemperatur nachgerührt. Dann wurden 20 l Wasser zudosiert, die obere organische Phase abgetrennt, die wäßrige Phase mit 2 l Methyl-tert.-butylether gewaschen, die vereinigten organischen Phasen mit 1 l 5%iger Salzsäure gewaschen und das Lösungsmittel abdestilliert. Man erhielt 4214 g der Titelverbindung in einer Reinheit von 96,6 % (GC-Flächenprozent) was einer Ausbeute von 89% entspricht.

Herstellung der Ketale III (Stufe 1)

### Beispiel 1

### 4,4-Dimethoxy-pentan-2,3-dion-3(E)-(O-methyloxim) (Tab.1, III.1)

4,3 g (0,03 mol) Pentan-2,3,4-trion-3-(O-methyloxim) und 6,2 g (0,06 mol) Trimethylorthoformiat wurden in 15 ml Methanol gelöst und mit einer Spatelspitze p-Toluolsulfonsäure versetzt. Anschließend wurde 5 h bei 50 °C gerührt und daraufhin das Lösungsmittel abdestilliert. Es wurden 5,5 g eines Öls (98% Ausbeute) erhalten (phys. Daten s. Tab. 1).

### Beispiel 2

### 1-(2-Methyl-[1,3]dioxolan-2-yl)-propan-1,2-dion-1(E)-(O-methyloxim) (Tab.1, III.2)

2400 g (39 mol) Ethylenglykol, 430 g (3,62 mol) Trimethylorthoformiat, 550 g (3,9 mol) Pentan-2,3,4-trion-3-(O-methyloxim) und 9 g p-Toluolsulfonsäure (46 mmol) wurden unter Rühren innerhalb von 15 min auf 85°C erhitzt. Nach 30 min bei 85°C wurde auf Raumtemperatur abgekühlt. Leichtflüchtige Anteile wurden während der Reaktion über einen Kolonnenkopf abdestilliert. Zur Aufarbeitung wurde mit gesättigter Natriumhydrogencarbonat-Lösung gewaschen, mit Methyl-tert.-butylether extrahiert, die vereinigten organischen Phasen zweimal mit Wasser gewaschen, und schließlich über Magnesiumsulfat getrocknet. Nach Abdestillieren des Lösungsmittels wurden 580 g rotbraunes Öl erhalten (phys. Daten s. Tab. 1).

### Beispiel 3

### 1-(2-Methyl-[1,3]dioxan-2-yl)-propan-1,2-dion-1(E)-(O-methyloxim) (Tab.1, III.3)

Ausgehend von 103 g (0,72 mol) Pentan-2,3,4-trion-3-(O-methyloxim), 275 g (3,62 mol) 1,3-Propandiol, 80 g (0,76 mol) Trimethylorthoformiat und 1,6 g p-Toluolsulfonsäure (9 mmol) wurden nach Vorschrift des Beispiels 2 137 g (94% Ausbeute) rötliches Öl erhalten, das nach HPLC eine Reinheit von 70% aufwies (phys. Daten s. Tab. 1).

### Beispiel 4

### 1-(2,5,5-Trimethyl-[1,3]dioxan-2-yl)-propan-1,2-dion-1(E)-(O-methyloxim) (Tab.1, III.4)

### a) mit Trimethylorthoformiat als wasserentziehendes Mittel

430 g (3 mol) Pentan-2,3,4-trion-3-(O-methyloxim), 1600 g (15 mol) Neopentylglykol, 330 g (3,15 mol) Trimethylorthoformiat und 7 g p-Toluolsulfonsäure wurden unter Rühren in 30 min auf 60 °C erhitzt. Nach 90 Minuten war die Reaktion beendet (DC- oder HPLC-Kontrolle). Zur Aufarbeitung wurde auf 20 °C abgekühlt und mit gesättigter Natriumbicarbonat-Lösung 15 min nachgerührt. Man gab 1 l Wasser zum Reaktionsgemisch und extrahierte mit Cyclohexan. Die organische Phase wurde einmal mit Wasser gewaschen, über Magnesiumsulfat getrocknet und eingeengt. Es wurden 663 g der Titelverbindung in einer Reinheit von 90% erhalten, was einer Ausbeute von 87% entspricht (phys. Daten s. Tab. 1).

### b) durch Wasserauskreisung (Schleppmittel: Cyclohexan)

100 g (0,69 mol) Pentan-2,3,4-trion-3-(O-methyloxim), 216 g (2,08 mol) Neopentylglykol und 0,25 g p-Toluolsulfonsäure wurden in 400 ml Cyclohexan zum Sieden erhitzt bis sich am Wasserabscheider kein Wasser mehr abtrennte (ca. 13 Stunden). Die Reaktionsmischung wurde auf Raumtemperatur abgekühlt und mit Wasser und Methylenchorid versetzt. Die organische Phase wurde über Natriumsulfat getrocknet. Nach Abdestillieren des Lösungsmittels verblieben 158,7 g eines Öls in einer Reinheit von 90% nach quant. HPLC, was einer Ausbeute von 90% entspricht.

### c) durch Wasserauskreisung (Schleppmittel: Toluol)

20 g (0,14 mol) Pentan-2,3,4-trion-3-(O-methyloxim), 43,4 g (0,42 mol) Neopentylglykol und 0,1 g konz. Schwefelsäure wurden in 80 ml Toluol 2 Stunden lang am Wasserabscheider bei 800mbar Druck zum Sieden erhitzt. Nach Extraktion mit Wasser und Toluol wurde wie in Beispiel 4b) aufgearbeitet. Erhalten wurden 30 g Öl in einer Reinheit von 84% (Ausbeute 79%).

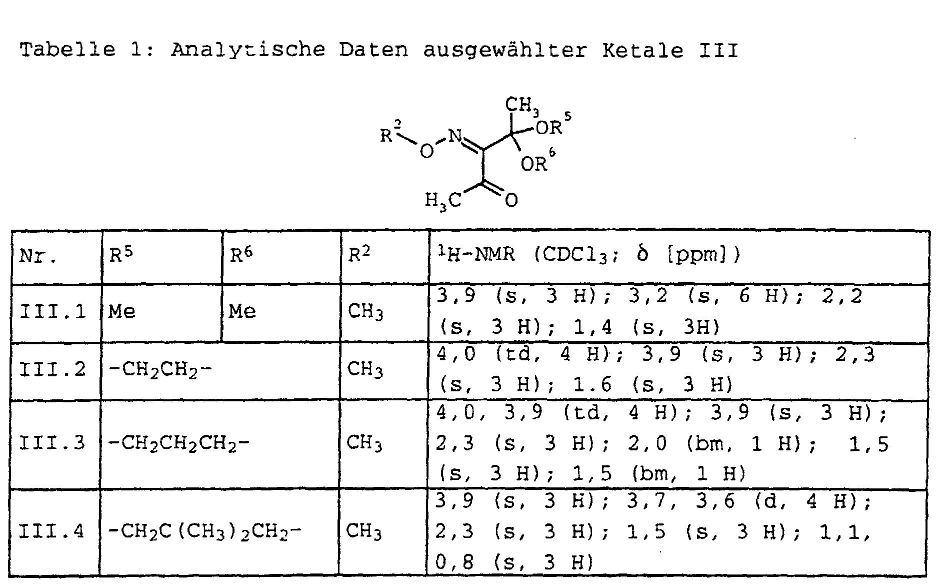

### Herstellung der Bisoximetherketale IV (Stufe 2)

### Beispiel 5

### 4,4-Dimethoxy-pentan-2,3(E,E)-dion-bis(O-methyloxim) (Tab.2, IV.1)

23 ml (0,3 mol) Pyridin und 2,5 g (0,03 mol) Methoxyaminhydrochlorid wurden bei Raumtemperatur vorgelegt. Anschließend wurden 5,5 g (0,03 mol) des Ketals (Beispiel 1), in 5 ml Methanol gelöst, zugetropft. Die Reaktionsmischung wurde ca. 18 Stunden bei Raumtemperatur gerührt. Zur Aufarbeitung wurde das Reaktionsgemisch eingeengt, in Methyl-tert.-butylether aufgenommen und nacheinander mit dest. Wasser, verd. HCl und Natriumbicarbonat-Lösung gewaschen. Nach Trocken über Magnesiumsulfat wurde das Lösungsmittel abdestilliert. Es wurden 4 g (61% Ausbeute) der Titelverbindung erhalten (phys. Daten der E,E-Isomeren s. Tab. 2).

### Beispiel 6

### 1-(2-Methyl-[1,3]dioxolan-2-yl)-propan-1,2-dion-bis(O-methyloxim) (Tab.2, IV.2)

Analog Beispiel 5 wurden aus 508 g (2,72 mol) des Ketals (Beispiel 2), 430 g (5,43 mol) Pyridin und 1570 g (2,72 mol) einer 14%igen Lösung von Methoxyaminhydrochlorid in Methanol 492 g der Titelverbindung erhalten (phys. Daten der E,E-Isomeren s. Tab. 2).

### Beispiel 7

### 1-(2-Methyl-[1,3]dioxan-2-yl)-propan-1,2-dion-bis(O-methyloxim) (Tab.2, IV.3)

### a) in Gegenwart von Pyridin/Methanol

Analog Beispiel 5 wurden aus 50 g (0,25 mol) des Ketals (Beispiel 3), 40 g (0,5 mol) Pyridin und 140 g (0,25 mol) einer 14%igen Lösung von Methoxyaminhydrochlorid in Methanol 46 g der Titelverbindung erhalten (phys. Daten der E,E-Isomeren s. Tab. 2).

### b) in Gegenwart von Pyridin/Wasser

45,5 g (0,2 mol) Propandiolketal (89%ig), 125,7 g Wasser, 40,9 g (0,517 mol) Pyridin und 134,7 g (0,484 mol) Methoxyaminhydrochlorid-Lösung (30%ig in Wasser) wurden 22 Stunden bei 25°C gerührt. Anschließend wurden 100 ml Methylenchlorid und 300 ml 2%ige Salzsäure hinzugegeben und die organische Phase abgetrennt. Die anorganische Phase wurde noch zweimal mit Methylenchlorid extrahiert. Die organischen Phasen wurden vereinigt, mit Wasser gewaschen und anschließend über Natriumsulfat getrocknet. Das Lösungsmittel wurde abdestilliert. Als Rückstand wurden 45,1 g der Titelverbindung mit einem Gehalt von 86,2% am EE-Isomeren und einer Ausbeute von 84,5% erhalten.

### c) in Gegenwart von Natriumacetat/Methanol

239 g (0,4 mol) 14%ige methanolische Methoxyamin-Lösung, 50 g (0,6 mol) Natriumacetat (wasserfrei), in 250 ml Methanol gelöst, 75 g Magnesiumsulfat und 92 g des Ketals (Beispiel 3), in 100 ml Methanol gelöst, wurden bei Raumtemperatur vorgelegt. Der pH-Meter zeigte einen Wert von 6 an. Es wurde 10 Minuten gerührt, dabei sank der pH auf 5,2, durch Zutropfen von Natriumacetat wurde ein pH von 4,2 eingestellt. Weitere 20 Stunden wurde bei Raumtemperatur nachgerührt und der Umsatz per HPLC kontrolliert. Es waren noch 7% Ausgangsmaterial vorhanden. Nach weiteren 4 Stunden Nachrühren wurde das Reaktionsgemisch mit verdünnter wäßriger Natronlauge neutralisiert und mit Wasser verdünnt. Es wurde mit Methyl-tert.-butylether extrahiert. Die vereinigten organischen Phasen wurden mit verdünnter Ammoniumchlorid-Lösung gewaschen, über Magnesiumsulfat getrocknet und eingeengt. Es wurden 92 g (89% Ausbeute) der Titelverbindung erhalten.

### d) in Gegenwart von Natriumacetat/Eisessig/Wasser

22,3 g (0,1 mol) Propandiolketal (90%ig), 61 g Wasser, 8,2 g Natriumacetat (0,1 mol) und 55,7 g (0,2 mol) Methoxyaminhydrochlorid-Lösung (30%ige Lösung in Wasser) wurden vorgelegt. Durch Zugabe von Eisessig wurde ein pH-Wert von 3,5 eingestellt. Anschließend rührte man 4 Stunden bei 25°C und gab dann 8,2 g (0,1 mol) Natriumacetat hinzu. Man rührte weitere 7 Stunden bei 25°C, fügte 50 ml Methylenchlorid hinzu und trennte die organische Phase ab. Die wäßrige Phase wurde dreimal mit Methylenchlorid extrahiert. Die vereinigten organischen Phasen wurden zweimal mit Wasser gewaschen und getrocknet. Nach dem Abdestillieren des Lösungsmittels verblieben 25 g der Titelverbindung mit einem Gehalt von 80,8% am EE-Isomeren und einer Ausbeute von 87,8%.

### Beispiel 8

### 1-(2,5,5-Trimethyl-[1,3]dioxan-2-yl)-propan-1,2-dion-bis (O-methyloxim) (Tab.2, IV.4)

### a) ausgehend von Beispiel 4

350 g (4,4 mol) Pyridin und 1,3 kg (2,2 mol) 14%ige methanolische Methoxyamin-hydrochlorid-Lösung wurden bei Raumtemperatur vorgelegt. 458 g (2,0 mol) des Ketals (Beispiel 4) wurden in 300 ml Methanol gelöst zugetropft und die Reaktionsmischung 18 Stunden gerührt. Nach Aufarbeitung analog Beispiel 5 wurden 484 g (93% Ausbeute) der Titelverbindung erhalten (phys. Daten der E,E-Isomeren s. Tab. 2).

### b) ausgehend von Pentan-2,3,4-trion-3-(O-methyloxim)

214,5 g (1,5 mol) Pentan-2,3,4-trion-3-(O-methyloxim), 2,75 g (0,0144 mol) p-Toluolsulfonsäure, 191 g (1,80 mol) Trimethylorthoformiat und 779,5 g (7,5 mol) Neopentylglykol wurden innerhalb von ca. 15 Minuten auf 85°C erwärmt. Man rührte 30 Minuten bei 85°C und kühlte anschließend auf 25°C ab. Die Reaktionsmischung wurde mit 215,8 g (2,84 mol) Pyridin und 1904 g (3,12 mol) Methoxyaminhydrochlorid-Lösung (13,7%ig in Methanol) versetzt und 24 Stunden bei 25°C gerührt. Man gab 2803 g Wasser hinzu, stellte durch Zugabe von 207 ml 50%iger Natronlauge einen pH-Wert von 7 ein und extrahierte dreimal mit Methyl-tert.-butylether. Die vereinigten organischen Phasen wurden zweimal mit 5%iger Salzsäure und anschließend mit Wasser gewaschen. Nach dem Trocknen über Natriumsulfat wurde das Lösungsmittel abdestilliert. Als Rückstand wurden 334 g der Titelverbindung mit einem Gehalt am EE-Isomeren von 78,4 % und einer Ausbeute über zwei Stufen von 67,6% erhalten.

### c) ausgehend von Beispiel 10

In einem Rührbehälter wurden bei 25°C 51,5 g Ketal-Oxim (Beispiel 10) und 221,5 ml DMF vorgelegt und mit 40,0 g (0,2 mol) 27%ige Natriummethylat-Lsg. versetzt. Man rührte 30 Minuten bei 25°C nach und destillierte anschließend das entstandene Methanol ab. Nun tropfte man 27,7 g (0,22 mol) Dimethylsulfat bei 20-25°C (Eiskühlung) zu und rührte 1 h bei 25°C nach. Danach wurde das Reaktionsgemisch am Rotationsverdampfer eingeengt. Der Rückstand (84.8g) wurde in 551,1 g Toluol, 33,6 g Wasser und 8,4g Dimethylamin-Lsg. (40%ig) aufgenommen u. 1,5 h bei Raumtemperatur gerührt.
Die Phasen wurden getrennt und die Wasser-Phase wurde mit Toluol extrahiert. Die vereinigten organischen Phasen wurden mit Wasser gewaschen und anschließend das Lösungsmittel bei reduziertem Druck abdestilliert. Es wurden 52,0g der Titelverbindung erhalten, was einer Ausbeute von 92% entspricht (laut quantitativen HPLC: 90,1% EE).

### Beispiel 9

### 1-(2-Methyl-[1,3]dioxolan-2-yl)-propan-1,2-dion-1-(O-methyloxim)-2-oxim (Tab.2, IV.5)

24 g (0,03 mol) 50%ige Natronlauge und 200 ml Wasser wurden bei 25°C vorgelegt und portionsweise mit insgesamt 25 g (0,0152 mol) Hydroxylammoniumsulfat versetzt. Dann wurden 50 g (0,0267 mol) des Ketals (Beispiel 2) zugetropft, und die Reaktionsmischung 9 Stunden bei 50°C (pH = 7- 8) gerührt. Danach wurde mit Natronlauge ein pH von 5 - 6 eingestellt und 48 Stunden bei 50°C gerührt. Es wurden nochmals 25 g Hydroxylammoniumsulfat und 24 g 50%ige Natronlauge zudosiert und weitere 20 Stunden bei 50°C gerührt. Anschließend wurden 300 ml Methyl-tert.-butylether hinzugegeben. Der im 2 Phasengemisch unlösliche Feststoff wurde abfiltriert, mit wenig Hexan gewaschen und getrocknet. Man erhielt 9 g der Titelverbindung (phys. Daten der E,E-Isomeren s. Tab. 2). Die organische Phase des als Mutterlauge erhaltenen 2-Phasengemischs wurde über Natriumsulfat getrocknet und anschließend am Rotationsverdampfer eingeengt. Es ergaben sich nochmals 17,5 g der Titelverbindung.

### Beispiel 10

### 1-(2,5,5-Trimethyl-[1,3]dioxan-2-yl)-propan-1,2-dion-1-(O-methyloxim)-2-oxim (Tab.2, IV.6)

In ähnlicher Weise wie oben beschrieben wurden aus 51 g Ketal (Beispiel 4) 57,7 g der Titelverbindung (Reinheit: ca. 90%) erhalten (phys. Daten der E,E-Isomeren s. Tab. 2).

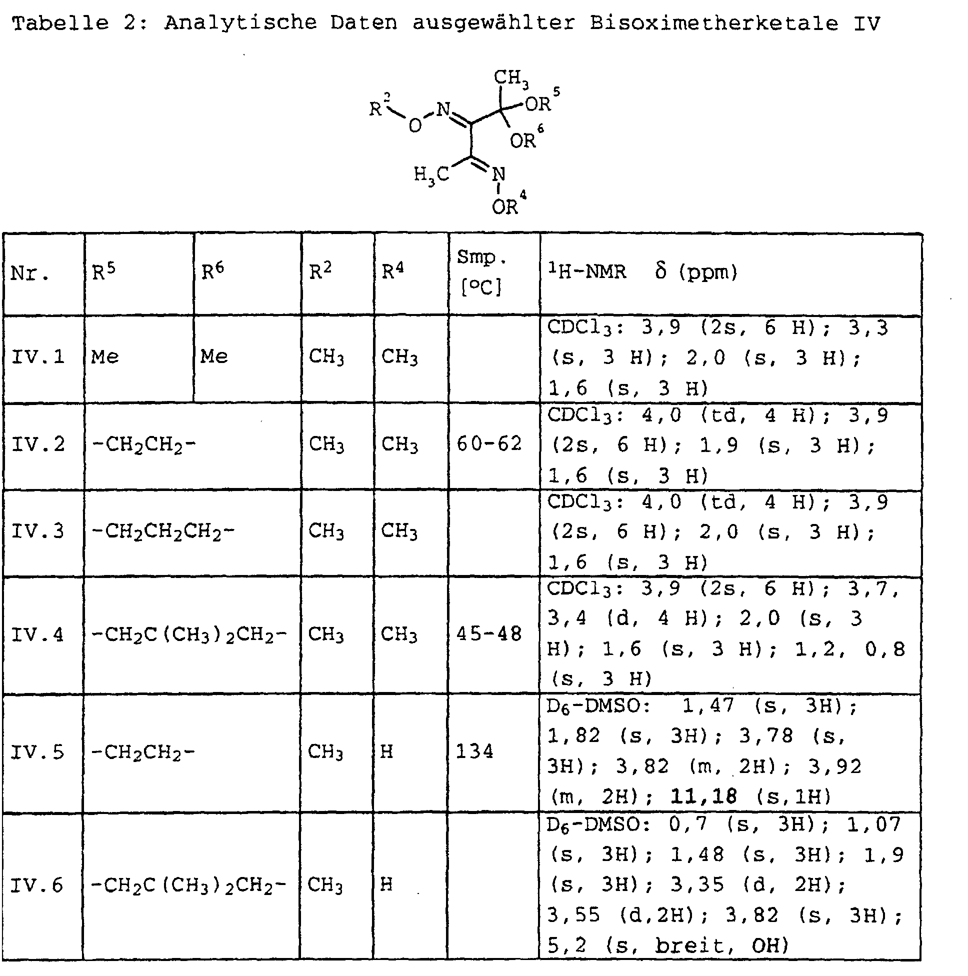

### Herstellung der Bisoximetheroxime Ia (Stufe 3a)

### Beispiel 11

### Pentan-2,3,4-trion-3,4-bis(O-methyloxim)

4,2 g des Beispiels 5 und 4,2 g Kieselgel 60 wurden in 10 ml Acetonitril gelöst. Man gab 10 ml Wasser und 3 Tropfen Trifluoressigsäure zu. Nach 30 Minuten wurde der gebildete Feststoff abgetrennt, das Filtrat mit Cyclohexan extrahiert und das Lösungsmittel abdestilliert. Man erhielt 1,7 g der Titelverbindung als Öl.
¹H-NMR (CDCl_{3,} δ [ppm]): 3,9 (2s, 6H), 2,3 (s, 3H), 2,0 (s, 3H).

### Herstellung der Bisoximetheroxime Ib (Stufe 3b)

### Beispiel 12

### Pentan-2,3,4-trion-3,4-bis(O-methyloxim)-2-oxim

### a) Oximierung mit Hydroxylammoniumchlorid

### aa) ausgehend von Beispiel 8

Zu 125 g Hydroxylammoniumchlorid in 500 ml Wasser wurden 387 g (1,5 mol) des Beispiels 8, in 500 ml Methanol gelöst, gegeben. Man gab noch 500 ml Eisessig zu und rührte 3 Stunden bei Raumtemperatur nach (HPLC-Kontrolle). Zur Aufarbeitung wurde das Reaktionsgemisch mit 20%iger Natronlauge unter Kühlung neutralisiert. Man extrahierte mit Methyl-tert.-butylether und entfernte das Lösungsmittel im Rotationsverdampfer. Das zurückbleibende Öl wurde in verd. Natronlauge aufgenommen und mit Methyl-tert.-butylether extrahiert. Die organische Phase wurde verworfen, die wässrige mit HCl angesäuert und mit Methyl-tert.-butylether extrahiert. Die organische Phase wurde über Magnesiumsulfat getrocknet und das Lösungsmittel im Roationsverdampfer abdestilliert.
Das zurückbleibende Öl kristallisierte beim Stehenlassen aus: 250 g (89% Ausbeute); Isomerenverhältnis: EZE/EZZ: 96 : 4.
¹H-NMR (CDCl₃; δ [ppm]): 1,92 (s, 3H); 2,12 (s, 3H); 3,92 (s, 3H); 3,99 (s, 3H); 9,92 (s, 1H);

### ab) ausgehend von Beispiel 7

45 g (0,2 mol) des Beispiels 7 (80%ige Reinheit) wurden in 100 ml Methanol gelöst. Es wurden 16 g (0,24 mol) Hydroxylammoniumchlorid in 100 ml Wasser gelöst, sowie 100 ml Eisessig zugegeben. Es bildete sich eine trübe Lösung, die 16 Stunden bis zum vollständigen Umsatz bei Raumtemperatur gerührt wurde (HPLC-Kontrolle). Zur Aufarbeitung wurde mit 50%iger Natronlauge neutralisiert, mit Methyl-tert.-butylether extrahiert und die organische Phase mit 2n NaOH gewaschen. Die NaOH-Phase wurde mit einem Gemisch aus Eis und Essigester versetzt und der pH mit konz. Salzsäure auf 2 eingestellt. Es wurde mit Essigester extrahiert, die organische Phase wurde mit gesättigter Natriumhydrogencarbonat-Lösung und mit wasser gewaschen. Es wurde über Magnesiumsulfat getrocknet, eingeengt und 29 g eines Öls erhalten; Isomerenverhältnis: EZE/EZZ: 96 : 4.

### ac) ausgehend von Beispiel 6

Analog zur Vorschrift 12 aa) wurden aus 216 g (1 mol) des Ketals (Beispiel 6), 139 g (2 mol) Hydroxylammoniumchlorid in einem Lösungsmittelgemisch von 500 ml THF, 500 ml Wasser und 500 ml Eisessig 125 g der Titelverbindung als farblose Kristalle erhalten, was einer Ausbeute von 67% entspricht.

### b) Oximierung mit Hydroxylammoniumsulfat

### ba) ausgehend von Beispiel 8

740 ml Wasser, 74 ml konz. Salzsäure, 148 ml Eisessig und 73,3 g (0,447 Mol) Hydroxylammoniumsulfat wurden bei 25°C mit einer Lösung von 297 g (0,739 Mol) des Beispiels 8 (roh; 64,2% EE-Isomer) in 740 ml Methanol versetzt. Man rührte 24 Stunden bei 25°C. Durch Zugabe von verd. NaOH wurde ein pH-Wert von 6 eingestellt und die Reaktionslösung zweimal mit Methyl-tert.-butylether extrahiert. Die vereinigten organischen Phasen wurden mit gesättigter NaHCO₃-Lösung gewaschen und getrocknet. Das Lösungsmittel wurde am Rotationsverdampfer abdestilliert. Isoliert wurden 190,9 g der Titelverbindung mit einem Isomerenverhältnis EZE : EZZ von 89,4 : 10,6. Die Ausbeute am EZE-Isomer betrug laut quantitativem HPLC 87,9%.

In weiteren Versuchen wurde gezeigt, daß auf die Anwesenheit des Cosolvens Eisessig verzichtet werden kann.

## Patentansprüche

1. Verfahren zur Herstellung von Trion-bis(oximether)derivaten der Formel I, 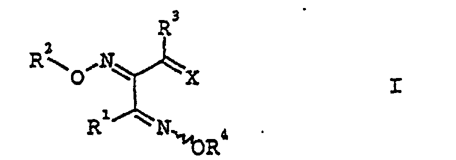 in der die Substituenten die folgende Bedeutung haben:
R¹,R³ unsubstituiertes, teilweise oder vollständig halogeniertes C₁-C₆-Alkyl oder C₃-C₆-Cycloalkyl;
R²,R⁴ unsubstituiertes C₁-C₄-Alkyl oder durch C₂-C₄-Alkenyl, C₂-C₄-Alkinyl oder Phenyl substituiertes Methyl und
X Sauerstoff oder N-OH,
**dadurch gekennzeichnet, daß** man
1) ein Dion der Formel II, 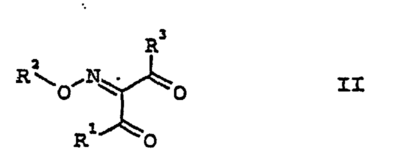 in der die Substituenten R¹, R² und R³ die vorgenannte Bedeutung besitzen, mit einem Alkohol oder Diol in Gegenwart einer Säure zum Ketal der Formel III, 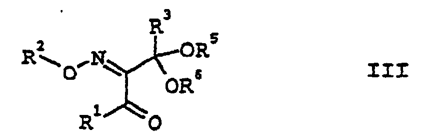 in der die Substituenten R⁵ und R⁶ C₁-C₆-Alkyl, Benzyl oder C₁-C₃-Haloalkyl bedeuten oder R⁵ und R⁶ zusammen mit dem Kohlenstoff- und den beiden Sauerstoffatomen der Ketalfunktion einen Ring A bilden, 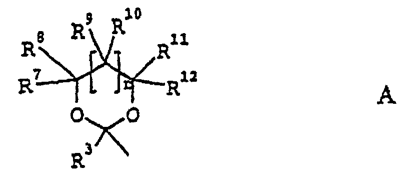 wobei die Substituenten und der Index n die folgende Bedeutung haben:
R⁷,R⁸,R¹¹,R¹² Wasserstoff, Halogen, C₁-C₄-Alkyl, C₁-C₃-Haloalkyl, C₁-C₄-Alkoxymethyl, C₂-C₄-Alkenyl, C₂-C₄-Alkinyl oder Phenyl, wobei letzeres durch Nitro oder Halogen substituiert sein kann;
R⁹,R¹⁰ eine der für R⁷, R⁸, R¹¹ oder R¹² gegebene Bedeutung sowie R⁹ und R¹⁰ gemeinsam eine exo-Methylen-Gruppe oder eine Carbonylgruppe und
n 0,1 oder 2,
umsetzt,
2) das so erhaltene Ketal III
a) mit einem Alkoxyamin der Formel R⁴O-NH₂, wobei R⁴ die vorgenannte Bedeutung besitzt, oder einem seiner Säureadditionssalze, oder
b) mit Hydroxylamin oder dessen Säureadditionssalz und anschließender Alkylierung mit einem Alkylierungsmittel R⁴-L¹, wobei R⁴ die vorgenannte Bedeutung besitzt und L¹ für eine nukleophil austauschbare Abgangsgruppe steht, in das Bisoximetherketal IV, 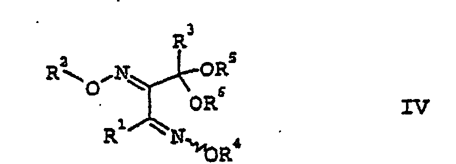
wobei die Substituenten R¹ bis R⁶ die vorgenannte Bedeutung besitzen, überführt und
3) das auf diese Weise erhaltene Bisoximetherketal IV in Gegenwart von Säure
a) zum Bisoximetherketon Ia, 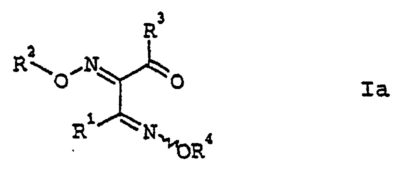 hydrolysiert oder
b) mit Hydroxylamin oder dessen Säureadditionssalz zum Bisoximetheroxim Ib, 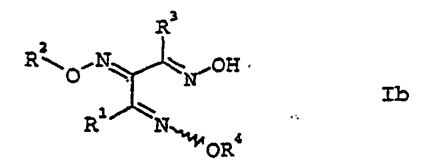
aminiert.

2. Verfahren gemäß Anspruch 1, **dadurch gekennzeichnet, daß** das Dion der Formel II in Stufe 1) mit einem Diol umgesetzt wird.

3. Verfahren gemäß Anspruch 2, **dadurch gekennzeichnet, daß** als Diol Ethylenglykol, 1,3-Propandiol oder 2,2-Dimethyl-1,3-propandiol eingesetzt werden.

4. Verfahren gemäß einem der Ansprüche 1 und 2, **dadurch gekennzeichnet, daß** in Stufe 2a) das Ketal III mit einem Säureadditionssalz des Alkoxyamins R⁴O-NH₂ bei Temperaturen von 20 bis 65°C umgesetzt wird und die bei der Reaktion freiwerdende Säure durch Zusatz von Basen mindestens teilweise gebunden wird.

5. verfahren gemäß einem der Ansprüche 1, 2 und 4, **dadurch gekennzeichnet, daß** in Stufe 3a)/3b) die Hydrolyse/Aminolyse bei einem pH von 0,5 bis 1.5 gestartet wird und bei Temperaturen von 20 - 40°C durchgeführt wird.

6. ketale der Formel III, 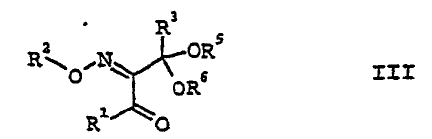 in der die Substituenten die folgende Bedeutung haben:
R¹,R³ unsubstituiertes, teilweise oder vollständig halogeniertes C₁-C₆-Alkyl oder C₃-C₆-Cycloalkyl;
R² unsubstituiertes C₁-C₄-Alkyl oder durch C₂-C₄-Alkenyl, C₂-C₄-Alkinyl oder Phenyl substituiertes Methyl;
R⁵,R⁶ C₁-C₆-Alkyl, Benzyl oder C₁-C₃-Haloalkyl oder
R⁵,R⁶ bilden zusammen mit dem Kohlenstoff- und den beiden Sauerstoffatomen der Ketalfunktion einen Ring A, 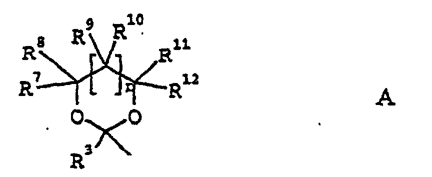 wobei
R⁷,R⁸,R¹¹,R¹² Wasserstoff, Halogen, C₁-C₄-Alkyl, C₁-C₃-Haloalkyl, C₁-C₄-Alkoxymethyl, C₂-C₄-Alkenyl, C₂-C₄-Alkinyl oder Phenyl, wobei letzeres durch Nitro oder Halogen substituiert sein kann;
R⁹,R¹⁰ eine der für R⁷, R⁸, R¹¹ oder R¹² gegebene Bedeutung sowie R⁹ und R¹⁰ gemeinsam eine exo-Methylen-Gruppe oder eine Carbonylgruppe und
n 0,1 oder 2 bedeuten.

7. Bisoximetherketale der Formel IV, 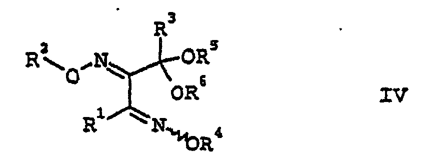 in der R⁴ Wasserstoff, unsubstituiertes C₁-C₄-Alkyl oder durch C₂-C₄-Alkenyl, C₂-C₄-Alkinyl oder Phenyl substituiertes Methyl bedeutet und die übrigen Substituenten die in Anspruch 6 gegebene Bedeutung besitzen.

8. Bisoximetherketale der Formel IV', 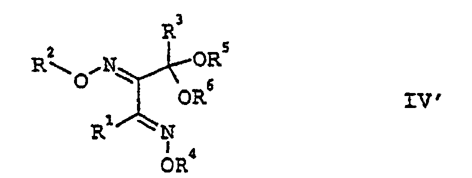 in der die substituenten R¹ bis R⁶ die in Anspruch 7 gegebene Bedeutung besitzen.

9. Verfahren gemäß Anspruch 1 zur Herstellung von Pentan-2,3,4-trion-3,4-bis (O-methyloxim).

## Claims

1. A process for preparing trione bis(oxime ether) derivatives of the formula I 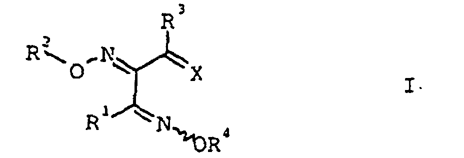 where the substituents have the following meanings:
R¹,R³ are each unsubstituted, partially or fully halogenated C₁-C₆-alkyl or C₃-C₆-cycloalkyl;
R²,R⁴ are each unsubstituted C₁-C₄-alkyl or C₂-C₄-alkenyl-, C₂-C₄-alkynyl- or phenyl-substituted methyl and
X is oxygen or N-OH,
which comprises
1) reacting a dione of the formula II, 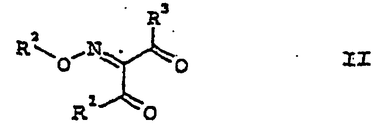 where the substituents R¹, R² and R³ are each as defined above with an alcohol or diol in the presence of an acid to give a ketal of the formula III, 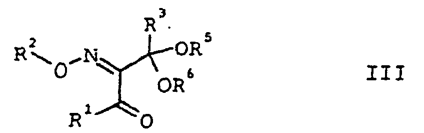 where the substituents R⁵ and R⁶ are each C₁-C₆-alkyl, benzyl or C₁-C₃-haloalkyl or R⁵ and R⁶ together with the carbon and the two oxygen atoms of the ketal function form a ring A 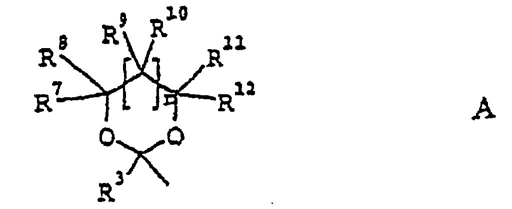 where the substituents and the index n have the following meanings:
R⁷,R⁸,R¹¹,R¹² are each hydrogen, halogen, C₁-C₄-alkyl, C₁-C₃-haloalkyl, C₁-C₄-alkoxymethyl, C₂-C₄-alkenyl, C₂-C₄-alkynyl or phenyl, where the latter may be substituted by nitro or halogen;
R⁹,R¹⁰ each have one of the meanings given for R⁷, R⁸, R¹¹ or R¹² and R⁹ and R¹⁰ together form an exo-methylene group or a carbonyl group and
n is 0,1 or 2,
2) converting the resulting ketal III
a) with an alkoxyamine of the formula R⁴O-NH₂, where R⁴ is as defined above, or one of its acid addition salts, or
b) with hydroxylamine or its acid addition salt and subsequent alkylation with an alkylating agent R⁴-L¹, where R⁴ is as defined above and L¹ is a nucleophilically replaceable leaving group, into the bisoxime ether ketal IV, 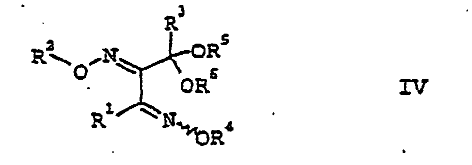
where the substituents R¹ to R⁶ are each as defined above, and
3) hydrolyzing the bisoxime ether ketal IV obtained in this manner in the presence of acid,
a) to give the bisoxime ether ketone Ia, 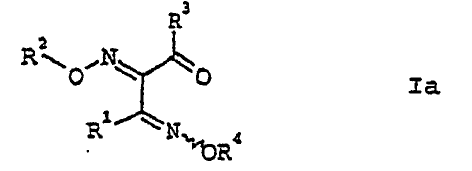 or
b) aminating it with hydroxylamine or its acid addition salt to give the bisoxime ether oxime Ib,

2. A process as claimed in claim 1 wherein the dione of the formula II is reacted with a diol in step 1).

3. A process as claimed in claim 2 wherein the diol employed is ethylene glycol, 1,3-propanediol or 2,2-dimethyl-1,3-propanediol.

4. A process as claimed in claim 1 or 2 wherein in step 2a) the ketal III is reacted with an acid addition salt of the alkoxyamine R⁴O-NH₂ at 20-65°C and the acid which is released during the reaction is at least partially bound by addition of bases.

5. A process as claimed in any of claims 1, 2 and 4 wherein in step 3a)/3b) the hydrolysis/aminolysis is started at a pH of from 0.5 to 1.5 and is carried out at 20 - 40°C.

6. A ketal of the formula III, 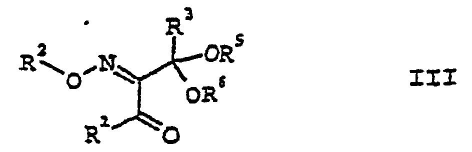 where the substituents have the following meanings:
R¹,R³ are each unsubstituted, partially or fully halogenated C₁-C₆-alkyl or C₃-C₆-cycloalkyl;
R² is unsubstituted C₁-C₄-alkyl or C₂-C₄-alkenyl-, C₂-C₄-alkynyl- or phenyl-substituted methyl;
R⁵,R⁶ are each C₁-C₆-alkyl, benzyl or C₁-C₃-haloalkyl or
R⁵,R⁶ together with the carbon and the two oxygen atoms of the ketal function form a ring A, 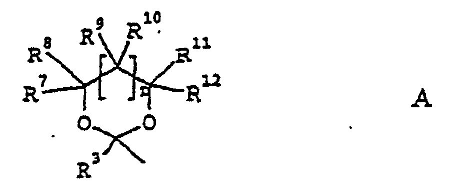
where
R⁷, R⁸, R¹¹, R¹² are each hydrogen, halogen, C₁-C₄-alkyl, C₁-C₃-haloalkyl, C₁-C₄-alkoxymethyl, C₂-C₄-alkenyl, C₂-C₄-alkynyl or phenyl, where the latter may be substituted by nitro or halogen;
R⁹,R¹⁰ each have one of the meanings given for R⁷, R⁸, R¹¹ or R¹² and R⁹ and R¹⁰ together form an exo-methylene group or a carbonyl group and
n is 0,1 or 2.

7. A bisoxime ether ketal of the formula IV, 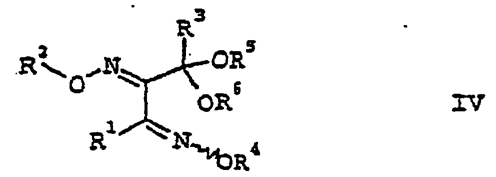 where R⁴ is hydrogen, unsubstituted C₁-C₄-alkyl or C₂-C₄-alkenyl-, C₂-C₄-alkynyl- or phenyl-substituted methyl and the other substituents are each as defined in claim 6.

8. A bisoxime ether ketal of the formula IV', 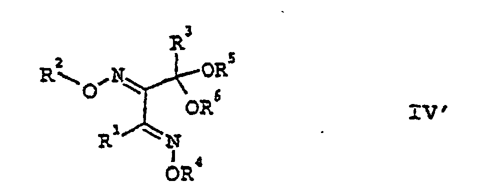 where the substituents R¹ to R⁶ are each as defined in claim 7.

9. A process as claimed in claim 1 for preparing pentane-2,3,4-trione 3,4-bis(O-methyloxime).

## Revendications

1. Procédé pour la préparation de dérivés de type trione-bis(oxime-éther) de formule I, 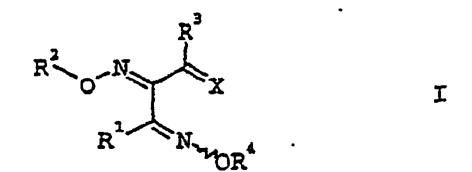 dans laquelle les substituants ont les significations suivantes :
R¹, R³ représentent un groupe alkyle en C₁-C₆ ou cycloalkyle en C₃-C₆ non substitué, partiellement ou totalement halogène ;
R², R⁴ représentent un groupe alkyle en C₁-C₄ ou un groupe méthyle substitué par un groupe alcényle en C₂-C₄, alcynyle en C₂-C₄ ou phényle et
X représente un atome d'oxygène ou N-OH,
**caractérisé en ce que**
1) on fait réagir une dione de formule II 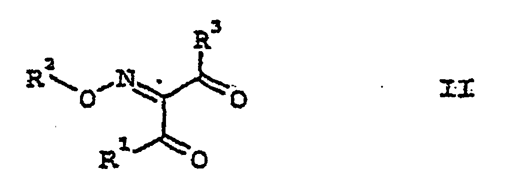 dans laquelle les substituants R¹, R² et R³ ont les significations données précédemment, avec un alcool ou un diol en présence d'un acide, pour aboutir au cétal de formule III 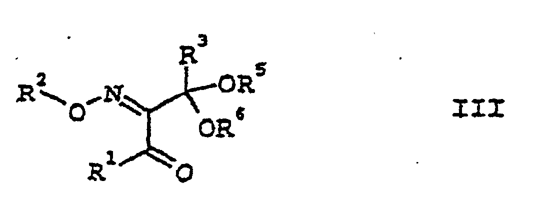 dans laquelle les substituants R⁵ et R⁶ représentent un groupe alkyle en C₁-C₆, benzyle ou halogénoalkyle en C₁-C₃, ou R⁵ et R⁶ forment ensemble, avec l'atome de carbone et les deux atomes d'oxygène de la fonction cétal, un cycle A, 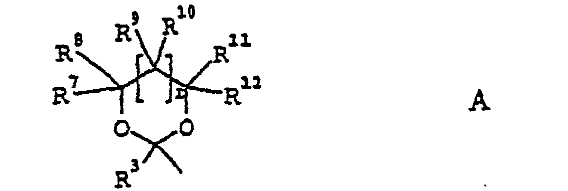 les substituants et l'indice n ayant les significations suivantes :
R⁷, R⁸, R¹¹, R¹² représentent un atome d'hydrogène ou d'halogène, ou un groupe alkyle en C₁-C₄, halogénoalkyle en C₁-C₃, alcoxy(C₁-C₄)méthyle, alcényle en C₂-C₄, alcynyle en C₂-C₄ ou phényle, ce dernier pouvant être substitué par un atome d'halogène ou par le groupe nitro ;
R⁹, R¹⁰ ont l'une des significations données pour R⁷, R⁸, R¹¹ ou R¹², ou R⁹ et R¹⁰ forment ensemble un groupe exométhylène ou un groupe carbonyle, et
n est 0, 1 ou 2,
2) on transforme le cétal III ainsi obtenu
a) avec une alcoxyamine de formule R⁴O-NH₂, R⁴ ayant la signification donnée précédemment, ou un de ses sels d'addition avec un acide, ou
b) avec de l'hydroxylamine ou un de ses sels d'addition avec un acide et par alkylation subséquente à l'aide d'un agent d'alkylation R⁴-L¹, R⁴ ayant la signification donnée précédemment et L¹ représentant un groupe partant échangeable par substitution nucléophile, en le bisoxime-éthercétal IV, 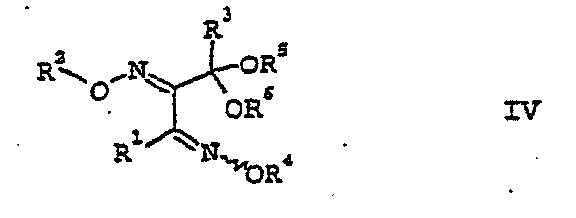 les substituants R¹ à R⁶ ayant les significations données précédemment, et
3) le bisoxime-éthercétal IV obtenu de cette façon, en présence d'acide
a) est hydrolysé en la bisoxime-éthercétone Ia, 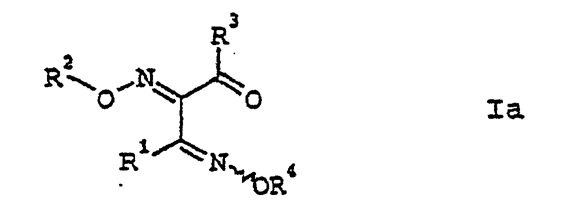 ou
b) est aminé avec de l'hydroxylamine ou un sel d'addition avec un acide de celle-ci, en la bisoxime-étheroxime Ib

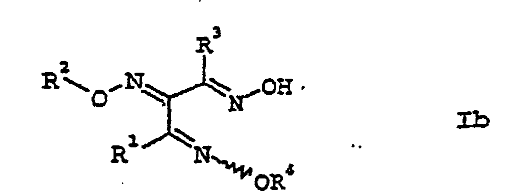

2. Procédé selon la revendication 1, **caractérisé en ce qu'**on fait réagir la dione de formule II dans l'étape 1) avec un diol.

3. Procédé selon la revendication 2, **caractérisé en ce qu'**on utilise comme diol l'éthylèneglycol, le 1,3-propanediol ou le 2,2-diméthyl-1,3-propanediol.

4. Procédé selon l'une quelconque des 1 ou 2, **caractérisé en ce que** dans l'étape 2a) on fait réagir le cétal III avec un sel d'addition avec un acide de l'alcoxyamine R⁴O-NH₂, à des températures de 20 à 65°C et l'acide libéré lors de la réaction est au moins en partie fixé par addition de bases.

5. Procédé selon l'une quelconque des revendications 1, 2 et 4, **caractérisé en ce que** dans l'étape 3a)/3b) l'hydrolyse/aminolyse est commencée à un pH de 0,5 à 1,5 et effectuée à des températures de 20-40°C.

6. Cétals de formule III 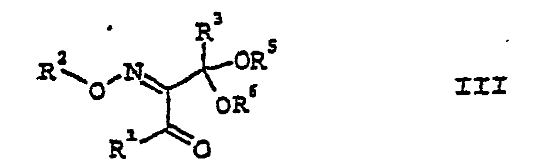 dans laquelle les substituants ont les significations suivantes :
R¹, R³ représentent un groupe alkyle en C₁-C₆ ou cycloalkyle en C₃-C₆ non substitué, partiellement ou totalement halogéné ;
R² représente un groupe alkyle en C₁-C₄ ou un groupe méthyle substitué par un groupe alcényle en C₂-C₄, alcynyle en C₂-C₄ ou phényle ;
R⁵, R⁶ représentent un groupe alkyle en C₁-C₆, benzyle ou halogénoalkyle en C₁-C₃ ou
R⁵, R⁶ forment ensemble, avec l'atome de carbone et les deux atomes d'oxygène de la fonction cétal, un cycle A, 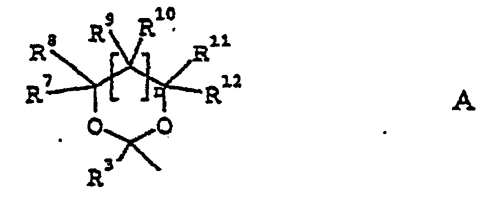 dans lequel
R⁷, R⁸, R¹¹, R¹² représentent un atome d'hydrogène ou d'halogène, ou un groupe alkyle en C₁-C₄, halogénoalkyle en C₁-C₃, alcoxy(C₁-C₄)méthyle, alcényle en C₂-C₄, alcynyle en C₂-C₄ ou phényle, ce dernier pouvant être substitué par un atome d'halogène ou par le groupe nitro ;
R⁹, R¹⁰ ont l'une des significations données pour R⁷, R⁸, R¹¹ ou R¹², ou R⁹ et R¹⁰ forment ensemble un groupe exométhylène ou un groupe carbonyle et
n est 0, 1 ou 2.

7. Bisoxime-éthercétals de formule IV 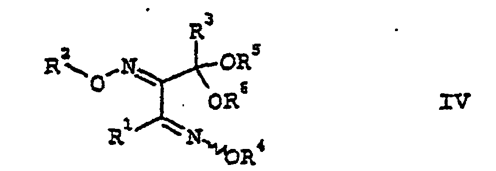 dans laquelle R⁴ représente un atome d'hydrogène, un groupe alkyle en C₁-C₄ non substitué ou un groupe méthyle substitué par un groupe alcényle en C₂-C₄, alcynyle en C₂-C₄ ou phényle, et les autres substituants ont les significations données dans la revendication 6.

8. Bisoxime-éthercétals de formule IV' 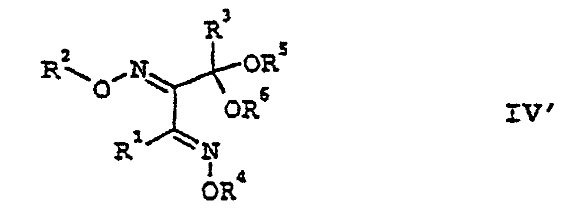 dans laquelle les substituants R¹ à R⁶ ont les significations données dans la revendication 6.

9. Procédé selon la revendication 1, pour la préparation de la pentane-2,3,4-trione-3,4-bis(O-méthyloxime).
